# FASCICULE DE BREVET EUROPEEN

(11) **EP 2 577 144 B1**
(45) Date de publication et mention de la délivrance du brevet: **02.10.2019**
(21) Numéro de dépôt: 11731436.9
(22) Date de dépôt: 20.04.2011
(51) Int. Cl.: F16L 37/04, F16L 33/30

(54) **RACCORDEMENT AVEC COMMUNICATION ENTRE CONTENANTS ET/OU CONDUITS BIOPHARMACEUTIQUES.**
VERBINDUNG MIT ÜBERTRAGUNG ZWISCHEN BIOPHARMAZEUTISCHEN BEHÄLTERN UND/ODER LEITUNGEN
JOINT WITH COMMUNICATION BETWEEN BIOPHARMACEUTICAL CONTAINERS AND/OR CONDUITS

(30) Priorité: 28.05.2010 FR 1054172
(43) Date de publication de la demande: 10.04.2013
(73) Titulaire: SARTORIUS STEDIM FMT SAS, 13400 Aubagne (FR)
(72) Inventeur: GUENERON, Maréva, F-13390 Auriol (FR)
(74) Mandataire: Derambure, Christian
(86) Numéro de dépôt international: PCT/FR2011/050916
(87) Numéro de publication internationale: WO 2011/148070

(56) Documents cités:
- EP-A2- 1 352 851
- EP-A2- 1 950 481
- DE-U1-202008 006 416
- FR-A1- 2 414 144
- US-A1- 2003 080 140
- US-A1- 2009 131 180
- US-B1- 6 653 377
- US-B1- 6 932 346

## Description

L'invention est relative aux raccordements (c'est-à-dire une liaison physique) avec communication entre contenants et/ou conduits biopharmaceutiques.

L'invention a plus particulièrement pour objet, en premier lieu, une pièce de raccordement avec communication pour la biopharmacie destinée à raccorder deux contenants et/ou conduits et mettre en communication leurs espaces intérieurs En deuxième lieu, un ensemble comprenant une telle pièce de raccordement avec communication et au moins une pince d'obturation et/ou au moins un moyen fonctionnel disposé au moins partiellement dans l'espace intérieur de la telle pièce. En troisième lieu, un assemblage constitué d'une telle pièce de raccordement avec communication, d'un premier contenant ou conduit et d'un second contenant ou conduit. En quatrième lieu, un dispositif fonctionnel pour la mise en oeuvre d'un processus biopharmaceutique comprenant au moins un premier contenant ou conduit et un second contenant ou conduit participant d'un tel assemblage. Et, en cinquième lieu un procédé pour raccorder avec communication en biopharmacie, deux contenants et/ou conduits

On entend ici par biopharmacie ou biopharmaceutique, ce qui est relatif à la biotechnologie, à la pharmacie et plus généralement au domaine médical. En particulier on entend par produit biopharmaceutique, un produit issu de la biotechnologie - milieux de cultures, cultures cellulaires, solutions tampon, liquides de nutrition artificielle, ou un produit pharmaceutique ou plus généralement un produit destiné à être utilisé dans le domaine médical, au moins pour partie sous forme solide plus ou moins finement divisé, sous forme liquide, ou sous forme pâteuse.

Il existe en biopharmacie le besoin de pouvoir raccorder avec communication de manière fixe, amovible et étanche, des pièces telles que des - ou des parties de - contenants et/ou conduits, qui, le cas échéant peuvent être intégrés dans des ensembles plus ou moins complexes pouvant comprendre plusieurs contenants et/ou conduits associés les uns avec les autres et le cas échéant un ou plusieurs moyens d'accès dans l'espace intérieur de tels contenants et/ou conduits et/ou un ou plusieurs moyens fonctionnels. De tels moyens fonctionnels sont par exemple aptes et destinés au traitement d'un produit biopharmaceutique, par exemple un moyen de mélange, d'aération, de mesure (collecte) de données, de pompage ou aptes et destinés au support des contenants et/ou conduits. De tels moyens fonctionnels sont, selon les cas, aptes et destinés à être placés en totalité à l'intérieur ou à l'extérieur ou pour partie à l'intérieur et pour partie à l'extérieur de tels contenants et/ou conduits.

Par convention, on désigne ici par «contenant », ce qui, en biopharmacie est apte et destiné dans son espace intérieur, à contenir, enfermer ou renfermer un certain contenu biopharmaceutique ou le cas échéant plusieurs contenus biopharmaceutiques, de façon statique plus ou moins durable ou pérenne. Un tel contenu biopharmaceutique est typiquement un produit biopharmaceutique mais peut être également un moyen fonctionnel, comme défini précédemment. De tels contenants sont par exemple des poches, des gaines, des conteneurs, des récipients, des bioréacteurs ou encore des goulottes à usage biopharmaceutique, cette liste n'étant pas limitative.

De tels contenants, considérés dans leur ensemble, sont soit rigides soit souples (par exemple pour pouvoir être pliés à plat avant usage) soit partiellement rigides et partiellement souples. Selon les cas, ils sont réalisés en acier inoxydable ou en matière plastique, ces réalisations n'étant pas limitatives, et leur contenance peut aller de moins d'un litre à plus de 3.000 litres, ces valeurs n'étant pas limitatives.

Selon les applications, de tels contenants ont une fonction de stockage, de transport, de manutention, de transfert ou encore de bioréaction, cette liste n'étant pas limitative.

Selon les cas de tels contenants sont à usage multiple ou à usage unique.

L'invention vise le cas de tels contenants du type comportant, notamment vers au moins une extrémité au moins une collerette annulaire résistante. Une telle collerette s'étend axialement vers l'extérieur du contenant. Une telle collerette comporte un renflement radial terminal s'étendant latéralement vers l'extérieur. Une telle collerette limite vers l'intérieur un orifice permettant l'accès à l'espace intérieur du contenant en vue notamment d'introduire dans le, ou d'extraire du, contenant, respectivement, le ou du certain contenu biopharmaceutique.

Des exemples de tels contenants sont décrits notamment dans les documents WO 2009/053572 et WO 2010/007273 et WO 2009/118494.

Par convention, on désigne ici par « conduit », ce qui, en biopharmacie est apte et destiné à canaliser ou guider un ou plusieurs contenus biopharmaceutiques, et plus spécialement produits biopharmaceutiques, dans son espace intérieur allongé, de manière à permettre son mouvement de l'amont vers l'aval. De tels conduits sont par exemple des tubes, des ports, des drains, des tuyaux, des gaines ou encore des goulottes à usage biopharmaceutique, cette liste n'étant pas limitative.

De tels conduits, considérés dans leur ensemble, sont soit rigides soit souples soit partiellement rigides et partiellement souples. Selon les cas, ils sont réalisés en acier inoxydable ou en matière plastique, Ces réalisations n'étant pas limitatives. Selon les cas, leur longueur et leur diamètre, respectivement, est plus ou moins grand.

Selon les applications, de tels conduits ont une fonction de transfert, mais ils peuvent également avoir une fonction de conservation par exemple d'échantillon, celte liste n'étant pas limitative.

Selon les cas, de tels conduits sont à usage multiple ou à usage unique.

L'invention vise le cas de tels conduits du type comportant à au moins une extrémité une collerette annulaire rigide. Une telle collerette s'étend axialement vers l'extérieur du conduit. Une telle collerette comporte un renflement radial terminal s'étendant latéralement vers l'extérieur. Une telle collerette limite vers l'intérieur l'orifice du conduit.

Il est entendu que le distinguo fait précédemment entre contenant et conduit vise essentiellement à illustrer chacun d'eux, non à les opposer, une goulotte, par exemple, pouvant être vue, selon le moment considéré et la fonction remplie, comme un contenant (assurant une fonction de maintien dans une localisation correspondant à l'espace intérieur de la goulotte) ou comme un conduit (assurant une fonction de canalisation dans l'espace intérieur de la goulotte). Toutefois, dans tous les cas, le contenant ou le conduit comporte au moins une collerette annulaire résistante s'étendant vers l'extérieur, comportant un renflement radial terminal s'étendant latéralement vers l'extérieur et limitant vers l'intérieur un orifice du contenant ou du conduit.

De façon traditionnelle et comme exposé dans la norme ISO 2852: 1993, il est prévu d'assembler ét de raccorder deux telles collerettes de manière fixe, amovible et étanche, au moyen d'une pince faisant fonction de bride, comprenant deux mors reliés entre eux, par exemple articulés, et des moyens de serrage et verrouillage. De telles pinces, également connues sous le nom de « tri-clamp » font l'objet de nombreuses variantes d'exécution, telles que celles décrites notamment dans les documents EP-A-1230505, US-A-6708377, US-A-7384078, cette liste n'étant pas limitative. Un tel tri-clamp comprend classiquement une menotte de serrage ayant à chacune de ses deux extrémités en correspondance un renflement, un organe de serrage coopérant structurellement et fonctionnellement avec les deux renflements radiaux latéraux en vis-à-vis du premier contenant ou conduit biopharmaceutique et du second contenant ou conduit biopharmaceutique. Plus précisément, la pince, bride ou tri-clamp est apte et destinée à venir se serrer sur le premier et le second renflements radiaux terminaux latéraux en les maintenant fermement l'un contre l'autre par leurs faces extrêmes transversales, pour éviter leur désassemblage intempestif et assurer l'étanchéité. Les documents EP-A-0997155 et EP-A-1352851 visent la mise en oeuvre de telles pinces ou tri-clamps.

Ces réalisations présentent un certain nombre de limites et d'inconvénients Ces tri-clamps, classiquement à usage multiple, sont coûteux Elles sont inadaptées au cas, de plus en plus fréquent et souhaité, d'un usage unique (en soi ou pour un processus pris globalement). Le plus souvent, leur diamètre intérieur ne dépasse pas une dizaine de centimètres, alors que de plus en plus fréquemment l'on envisage l'usage de dispositifs biopharmaceutiques plus importants en taille, notamment s'agissant des orifices des contenants. Leur mise en place s'avère souvent malaisée, longue et hasardeuse, dans la mesure où l'opérateur doit tenir la tri-clamp et la manoeuvrer (pour la fermer) et simultanément tenir au moins l'un du premier contenant ou conduit et du second contenant ou conduit, voire les deux, tout en les maintenant parfaitement positionnées l'un par rapport à l'autre. Ces inconvénients sont rédhibitoires lorsque, comme on le souhaite de plus en plus souvent, les deux pièces doivent pouvoir être assemblées rapidement et plus facilement.

En outre, ces réalisations ne permettent que l'assemblage d'un premier contenant ou conduit biopharmaceutique et d'un second contenant ou conduit biopharmaceutique, dont les orifices respectifs ont des diamètres identiques.

Le document US-A-2003/0080140 décrit une poche, ici de petite contenance, ayant un port de sortie, sur lequel est monté un connecteur sur lequel est monté à son tour un tube.

Le document DE 20 2008 006416 U1 décrit une sortie en forme de collerette avec une saillie annulaire sur laquelle est monté un tube.

Le document EP-A-1950481 décrit un tuyau en silicone et un connecteur réutilisable pour connecter une extrémité de tuyau à un tel tuyau en silicone.

Le document US-6653377 décrit un système pour le transfert de particules utilisant un ensemble de cartouche à joints toriques multiples qui peut être monté soit sur le port de déchargement ou de port de chargement d'une pièce d'équipement de transformation.

Les dispositifs objet de ces quatre derniers documents sont de portée générale mais ne visent pas l'application plus spécialement visée par l'invention.

Par conséquent, il existe, dans le domaine biopharmaceutique, le besoin de pouvoir raccorder avec communication, de manière fixe, amovible et étanche, une première collerette et une seconde collerette telles que décrites précédemment, appartenant à un premier contenant ou conduit biopharmaceutique et à un second contenant ou conduit biopharmaceutique, respectivement, de manière à assurer une communication entre le premier orifice et le second orifice, respectivement, du premier contenant ou conduit biopharmaceutique et du second contenant ou conduit biopharmaceutique.

Plus spécifiquement, il existe ce besoin avec comme exigences la possibilité d'un usage unique (en soi ou pour un processus pris globalement), un faible coût, une mise en place (c'est-à-dire montage ou assemblage) et un désassemblage rapides et sûres, un montage ou assemblage étanche, une possibilité d'application dans le cas d'un premier orifice et d'un second orifice ayant des diamètres différents, pouvant atteindre une cinquantaine de centimètres.

Egalement, il existe en biopharmacie le besoin de disposer de dispositifs fonctionnels du type comprenant un premier contenant ou conduit biopharmaceutique et un second contenant ou conduit biopharmaceutique raccordés entre eux avec communication, de manière à répondre aux exigences précédemment mentionnées, notamment procédant d'une conception « versatile » quant aux applications, aux fonctions remplies et aux diamètres des orifices. S'agissant du domaine biopharmaceutique, il importe également que les matériaux mis en oeuvre soient tels que les composants et dispositifs réalisés à partir d'eux gardent leur intégrité lors de la stérilisation, par exemple par rayonnement γ, que les dispositifs mis en oeuvre satisfassent aux agréments des autorités sanitaires et soient compatibles avec une utilisation en salle blanche.

L'invention a pour but de répondre à ces besoins rencontrés en biopharmacie.

A cet effet, selon un premier aspect, l'invention a pour objet une pièce de raccordement selon la revendication 1.

Selon une réalisation, la première ouverture de communication et la seconde ouverture de communication ont des diamètres différents. En particulier, la première ouverture de communication et la seconde ouverture de communication ont des diamètres différents dans un rapport allant de 1 à 10.

Selon une réalisation, la pièce de raccordement avec communication est apte à se trouver dans une configuration aplatie d'obturation transversale en au moins une zone transversale d'obturation où les zones en vis-à-vis de la face intérieure de la gaine sont appliquées l'une contre l'autre et empêchent une communication de part et d'autre d'une telle zone transversale d'obturation.

Selon une réalisation, la pièce de raccordement avec communication comporte une unique première et/ou seconde partie extrême libre et une unique première et/ou seconde jupe de raccordement, la pièce de raccordement avec communication étant destinée à être raccordée avec communication à une unique première et/ou seconde collerette d'un unique premier et/ou second contenant ou conduit.

Selon une réalisation, la gaine et les deux jupes de raccordement sont en un matériau intrinsèquement antistatique ou en un matériau rendu antistatique extrinsèquement.

Selon une réalisation, la gaine et les deux jupes de raccordement sont en silicone ou comprennent du silicone.

Selon une réalisation, la pièce de raccordement avec communication pour la biopharmacie est à usage unique.

Selon un deuxième aspect, l'invention à pour objet un ensemble comprenant une pièce de raccordement avec communication pour la biopharmacie telle qu'elle vient d'être décrite et au moins une pince d'obturation apte à se trouver soit dans une configuration ouverte inactive soit dans une configuration fermée dans laquelle elle est apte à venir se placer autour de la gaine et s'appliquer sur les zones en vis-à-vis de sa face extérieure dans au moins une zone transversale d'obturation, de sorte que les zones en vis-à-vis de la face intérieure de la gaine sont appliquées l'une contre l'autre et empêchent une communication de part et d'autre d'une telle zone transversale d'obturation.

Selon un deuxième aspect, l'invention a pour objet un ensemble comprenant une pièce de raccordement avec communication pour la biopharmacie telle qu'elle vient d'être décrite et au moins un moyen fonctionnel disposé au moins partiellement dans l'espace intérieur de raccordement.

Selon un troisième aspect, l'invention a pour objet un assemblage constitué d'une pièce de raccordement avec communication pour la biopharmacie telle qu'elle vient d'être décrite, d'un premier contenant ou conduit et d'un second contenant ou conduit, tel que :
▪ la face latérale intérieure de la première jupe de raccordement est au contact avec serrage élastique de maintien et étanchéité sur une partie conjuguée de la face extérieure de la première collerette, sans la nécessité d'une pièce extrinsèque de fixation,
▪ et la face latérale intérieure de la seconde jupe de raccordement est au contact avec serrage élastique de maintien et étanchéité sur une partie conjuguée de la face extérieure de la seconde collerette, sans la nécessité d'une pièce extrinsèque de fixation.

Selon une réalisation, l'assemblage comporte un unique premier et/ou second contenant ou conduit et une pièce de raccordement avec communication pour la biopharmacie dans la réalisation correspondante de cette pièce.

Selon une réalisation, l'assemblage comporte en outre au moins un moyen fonctionnel disposé au moins partiellement dans l'espace intérieur de raccordement.

Selon un quatrième aspect, l'invention a pour objet un dispositif fonctionnel pour la mise en oeuvre d'un processus biopharmaceutique comprenant au moins un premier contenant ou conduit et un second contenant ou conduit devant être raccordés avec communication, dans lequel le premier contenant ou conduit, le second contenant ou conduit et une pièce de raccordement avec communication pour la biopharmacie telle qu'elle vient d'être décrite forment un assemblage tel qu'il vient d'être décrit.

Selon un cinquième aspect, l'invention a pour objet un procédé pour raccorder avec communication en biopharmacie, deux contenants et/ou conduits comportant, respectivement, une première collerette annulaire résistante et une seconde collerette annulaire résistante limitant un premier orifice et un second orifice, caractérisé par le fait que :
▪ on dispose du premier contenant ou conduit et du second contenant ou conduit,
▪ on dispose d'une pièce de raccordement avec communication pour la biopharmacie telle qu'elle vient d'être décrite se trouvant à l'état désassemblé,
▪ on étire transversalement la pièce de raccordement avec communication à l'endroit de ses deux jupes de raccordement jusqu'à être élargie transversalement pour pouvoir être enfilée sur les deux collerettes,
▪ puis, on laisse la pièce de raccordement avec communication venir, par son élasticité, au contact avec serrage élastique de maintien et étanchéité sur la partie conjuguée de la face extérieure des deux collerettes et être ainsi amenée à l'état assemblé, sans la nécessité d'une pièce extrinsèque de fixation.

D'autres caractéristiques et avantages ressortiront clairement de la description qui en est faite ci-après, à titre indicatif et nullement limitatif, en référence aux dessins annexés, dans lesquels :
- la figure 1 est une représentation en perspective d'un assemblage composé d'une pièce de raccordement selon l'invention permettant la communication entre un premier contenant et un second contenant ;
- la figure 2 est une représentation en coupe de l'assemblage de la figure 1 dans laquelle la première collerette du premier contenant est espacée dans la direction verticale de la seconde collerette du second contenant ;
- la figure 3 est une représentation en coupe de l'assemblage de la figure 1 dans laquelle la première collerette du premier contenant est partiellement introduite dans l'espace interne défini par la seconde collerette du second contenant ;
- la figure 4 est une représentation en coupe de l'assemblage de la figure 1 dans laquelle un outil d'obturation est utilisé pour maintenir la pièce de raccordement dans une configuration fermée en une zone transversale d'obturation ;
- la figure 5 est une représentation en coupe d'un assemblage composé d'une pièce de raccordement permettant la communication entre deux premiers contenants et un second contenant ;
- la figure 6 est une représentation en coupe d'un assemblage composé d'une pièce de raccordement permettant la communication entre un premier contenant et deux seconds contenants ;
- la figure 7 est une représentation en coupe d'un assemblage composé d'une pièce de raccordement permettant la communication entre un premier contenant et un second contenant

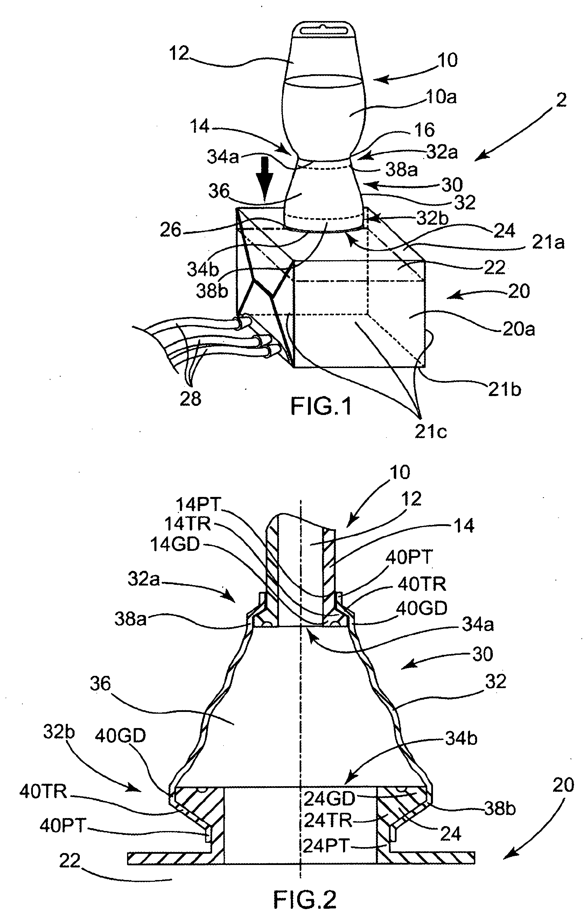

La figure 1 est une vue en perspective d'un assemblage 2.

Cet assemblage 2 est composé d'un premier contenant 10 formant une poche souple transparente et hermétique capable de contenir un produit amont 10a biopharmaceutique au moins pour partie sous forme solide plus ou moins finement divisé, sous forme liquide, ou sous forme pâteuse (milieux de cultures, cultures cellulaires, solutions tampon, liquides de nutrition artificielle, etc.).

En l'espèce, ce premier contenant 10 forme une poche souple transparente. Toutefois, ce premier contenant 10 pourrait également présenter une forme différente, réalisée à partir d'un matériau solide et non transparent.

Ce premier contenant 10 forme un premier espace intérieur 12 et comporte une extrémité sur laquelle est formée une première collerette annulaire 14 limitant un premier orifice 16.

L'assemblage 2 de la figure 1 comporte également un second contenant 20 formant une poche 3D solide et hermétique à l'intérieur de laquelle est défini un second espace intérieur 22 capable de contenir un produit aval 20a tel qu'un liquide, un fluide, ou tout autre type de produit biopharmaceutique, pharmaceutique ou médicale.

Ce second contenant 20 comporte une surface supérieure 21 a sur laquelle est formée une seconde collerette annulaire 24 limitant un second orifice 26. Le second contenant 20 présente, par ailleurs, une surface inférieure 21b opposée à la surface supérieure 21a ainsi que quatre surfaces latérales 21c reliant les surfaces supérieure 21a et inférieure 21b. L'une des quatre surfaces latérales 21c comporte une pluralité d'orifices de communication apte à se connecter à l'extrémité de tuyaux de transfert 28 afin de réaliser notamment un transfert du produit aval 20a vers des contenants secondaires.

Selon un mode de réalisation non limitatif, ces tuyaux de transfert 28 peuvent en outre former un circuit fermé dotée d'une pompe péristaltique permettant de faire circuler et de mélanger continuellement le produit aval 20a.

Comme précédemment le second contenant 20 peut également présenter une forme différente de celle illustrée sur la figure 1. Plus particulièrement, s'il est fait référence à des contenants 10, 20 en référence aux figures illustratives 1 à 7, il convient de rappeler que l'invention n'est pas limitée au raccordement de tels contenants mais s'étend au contraire au raccordement de tout type de conduits tel précédemment cités.

L'assemblage 2 de la figure 1 comporte en outre une pièce de raccordement 30 comportant une gaine de raccordement 32 fermée latéralement sur elle-même. La gaine de raccordement 32 est pourvue à une première partie extrême libre 32a d'une première ouverture de communication 34a et, à une seconde partie extrême libre 32b, d'une seconde ouverture de communication 34b. Entre ses première et seconde ouvertures de communication 34a, 34b la gaine de raccordement 32 est apte à former et limiter un espace intérieur de raccordement 36.

Avantageusement, la gaine de raccordement 32 présente, à l'état assemblée et en configuration de communication, une forme générale cylindrique ou tronconique de section transversale au moins sensiblement circulaire ou pseudo-circulaire.

Elle présente en outre, une face intérieure substantiellement sans aspérité, relief ou rugosité. Cette face intérieure est plus spécialement lisse afin d'une part, de ne pas freiner le transfert du produit amont 10a vers le seconde contenant 20 et, d'autre part, de ne pas conserver de ce produit amont 10a à l'intérieur de la pièce de raccordement 30.

A la première partie extrême libre 32a de la gaine de raccordement 32 est formée une première jupe extérieure de raccordement 38a, annulaire et périphérique, dont la face latérale intérieure est apte à venir au contact avec serrage élastique de maintien et étanchéité sur une partie conjuguée de la face exténeure de la première collerette du premier contenant 10.

A la seconde partie extrême libre 32b de la gaine de raccordement 32 est formée une seconde jupe extérieure de raccordement 38b, annulaire et périphérique, dont la face latérale intérieure est apte à venir au contact avec serrage élastique de maintien et étanchéité sur une partie conjuguée de la face extérieure de la seconde collerette 24 du second contenant 20.

Ainsi, comme l'illustre la figure 1, la première partie extrême libre 32a de la pièce de raccordement 32 peut être raccordée à la première collerette annulaire 16 du premier contenant 10 et la seconde partie extrême libre 32b de la gaine de raccordement 32 peut être raccordée à la seconde collerette annulaire 26.

Il convient de signaler que la gaine de raccordement 32 et les première et seconde jupes de raccordement 38a, 38b sont d'un seul tenant et formées dans un matériau intrinsèquement étanche pour la biopharmacie. Cette gaine de raccordement 32 et ces première et seconde jupes 38a, 38b présentent une élasticité telle que la pièce de raccordement 30 est apte, d'une part, à partir d'un état désassemblé, à être étirée transversalement à l'endroit de ses deux jupes de raccordement 38a, 38b jusqu'à être élargie transversalement, sans perte d'intégrité, pour être rendue apte à être enfilée sur les deux collerettes annulaires 14, 24 des premier et second contenant 10, 20 puis - par son élasticité - venir au contact avec serrage élastique de maintien et étanchéité sur la partie conjuguée de la face extérieure des deux collerettes annulaires 16, 26. Ainsi, la pièce de raccordement 30 peut être amenée à l'état assemblé, sans la nécessité d'une pièce extrinsèque de fixation. En outre, la gaine de raccordement 32 et les première et seconde jupe de raccordement 38a, 38b présentent une élasticité telle que la pièce de raccordement 30 est apte, d'autre part, à partir de l'état assemblé, à être étirée transversalement à l'endroit de ses deux jupes de raccordement 38a, 38b jusqu'à être élargie transversalement pour pouvoir être désenfilée des deux collerettes annulaires 16, 26 et être ainsi amenée à l'état désassemblé.

Afin de raccorder avec communication les premier et second contenants 10, 20 :
▪ on dispose du premier contenant 10 et du second contenant 20,
▪ on dispose d'une pièce de raccordement 30 avec communication se trouvant à l'état désassemblé,
▪ on étire transversalement la pièce de raccordement 30 avec communication à l'endroit de ses deux jupes de raccordement 38a, 38b successivement ou simultanément - jusqu'à être élargie transversalement pour pouvoir être enfilée respectivement sur les première et seconde collerettes 16, 26,
▪ puis, on laisse la pièce de raccordement 30 venir, par son élasticité, au contact avec serrage élastique de maintien et étanchéité sur la partie conjuguée de la face extérieure des deux collerettes 16, 26 et être ainsi amenée à l'état assemblé, sans la nécessité d'une pièce extrinsèque de fixation.

De cette façon, la face latérale intérieure de la première jupe de raccordement 38a est au contact avec serrage élastique de maintien et étanchéité sur une partie conjuguée de la face extérieure de la première collerette annulaire 16, sans la nécessité d'une pièce extrinsèque de fixation, et la face latérale intérieure de la seconde jupe de raccordement 38b est au contact avec serrage élastique de maintien et étanchéité sur une partie conjuguée de la face extérieure de la seconde collerette annulaire 26, sans la nécessité d'une pièce extrinsèque de fixation.

Par ailleurs, la pièce de raccordement 30 est apte, à l'état assemblé et dans la configuration de communication, à permettre la localisation et/ou la canalisation dans son espace intérieur d'au moins une partie du produit amont 10a originellement situé dans l'espace intérieur du premier contenant 10.

Il convient de souligner que, selon la réalisation de la figure 1, la gaine de raccordement 32 et les première et secondé jupes de raccordement 38a, 38b sont d'un seul tenant de fabrication. Cela permet d'assurer un confinement optimal à l'intérieur de l'espace intérieur de raccordement 36, sans risque de fuites liées à l'assemblage de la gaine de raccordement 32 avec les première et seconde jupes de raccordement 38a, 38b.

Toutefois, il pourrait également être envisage que l'une ou l'autre des première et seconde jupes de raccordement 38a, 38b - voir les deux - soit raccordée,(s) à la gaine de raccordement 32 par collage, soudage, couture ou tout autre moyen de fixation permettant d'assurer une liaison suffisamment hermétique pour la communication de produits biopharmaceutiques.

D'autre part, selon une réalisation la gaine de raccordement 32 et les première et seconde jupes de raccordement 38a, 38b sont en un matériau intrinsèquement antistatique ou en un matériau rendu antistatique extrinsèquement. Ainsi, le passage du produit biopharmaceutique, notamment lorsqu'il est poudreux à l'intérieur de l'espace intérieur de raccordement 36 n'est pas ralenti du fait de la paroi interne de la gaine de raccordement 32.

En outre, selon une réalisation la gaine de raccordement 32 et les première et seconde jupes de raccordement 38a, 38b sont en silicone ou comprennent en partie du silicone.

Le premier contenant 10, le second contenant 20 et/ou la pièce de raccordement 30 peuvent être à usage unique, ce qui permet d'éliminer toute étape de nettoyage après que le produit amont 10a ait été déversé dans le second contenant 20. par exemple.

La figure 2 est maintenant décrite en détail.

Celle-ci représente, en coupe verticale, l'assemblage 2 de la figure 1 et précise la structure de la première collerette annulaire 14 et de la seconde collerette annulaire 24.

Plus particulièrement, celles-là présentent des caractéristiques mécaniques telles qu'il leur est possible d'assumer les pressions exercées respectivement par la première partie extrême libre 32a et la seconde partie extrême libre 32b de la gaine de raccordement 32, sans subir de modification substantielle de leur géométrie. Sans cela le caractère hermétique de la communication entre le premier contenant 10 et le second contenant 20 pourrait être altéré.

Selon cette réalisation, la face latérale intérieure de l'une et l'autre des première et seconde jupes de raccordement 38a, 38b comportent une zone cylindrique de plus grand diamètre intérieur 40GD attenante à la gaine de raccordement 32, une zone cylindrique de plus petit diamètre intérieur 40PT attenante à la partie extrême libre 32a, 32b de la pièce de raccordement 30 et une zone tronconique 40TR placée entre la zone cylindrique de plus grand diamètre intérieur 40GR et la zone cylindrique de plus petit diamètre intérieur 40PT.

De la même façon, la surface extérieure de l'une et l'autre des première et seconde collerettes annulaires 14, 24 comportent une zone cylindrique de plus grand diamètre extérieur 14GR, 24GR proche de leur chant d'extrémité, une zone cylindrique de plus petit diamètre extérieur 14PT, 24PT et une zone tronconique 14TR, 24TR placée entre la zone cylindrique de plus grand diamètre 14GD, 24GD et la zone cylindrique du plus petit diamètre extérieur 14PT, 24PT des première et seconde collerettes annulaires 14, 24.

Ainsi, le maintien des première et seconde jupes de raccordement 38a, 38b sur les première et seconde collerettes annulaires 14, 24, respectivement est assuré non seulement par l'élasticité du matériau formant la pièce de raccordement 30 mais également par la forme complémentaire de ces première et seconde jupes de raccordement 38a, 38b avec les première et seconde collerettes annulaires 14, 24.

La pression exercée par la zone tronconique 40TR des première et seconde jupes de raccordement 38a, 38b sur la zone tronconique 14TR, 24TR des première et seconde collerettes annulaires 14, 24 est ainsi amplifiée lorsque la première jupe de raccordement 38a est éloignée de la seconde jupe de raccordement 38b.

Le caractère hermétique de l'assemblage 2 formé notamment par la pièce de raccordement 30 est ainsi augmenté.

Selon la réalisation de la figure 2, la face latérale extérieure de l'une et l'autre des jupes de raccordement 38a, 38b comportent également une zone cylindrique de plus grand diamètre extérieur 40GR attenante à la gaine de raccordement 32, une zone cylindrique de plus petit diamètre extérieur 40PT attenante au bord libre d'extrémité de la pièce de raccordement 30 et une zone tronconique 40TR placée entre la zone cylindrique de plus grand diamètre extérieur 40GD et la zone cylindrique de plus petit diamètre extérieur 40PT.

De cette façon, la première jupe de raccordement 38a et la seconde jupe de raccordement 38b comprennent une épaisseur radiale identique ou sensiblement identique dans la zone cylindrique de plus grand diamètre 40GR, dans la zone cylindrique de plus petit diamètre 40PT et dans la zone tronconique 40TR. Cela permet simplifier la géométrie de la pièce de raccordement 30 et donc de faciliter sa fabrication.

Selon une réalisation alternative, ces première et seconde jupes de raccordement 38a, 38b pourraient toutefois présenter une épaisseur radiale plus grande dans la zone cylindrique de plus grand diamètre 40GR que dans la zone cylindrique de plus petit diamètre 40PT et dans la zone tronconique 40TR. Une telle réalisation permet avantageuse de renforcer les propriétés mécaniques des première et seconde jupes de raccordement 38a, 38b dans cette zone cylindrique de plus grand diamètre 40GR afin de permettre son étirement sans risquer de l'endommager.

Il convient également de souligner que, selon la réalisation illustrée par cette figure 2, l'une et l'autre des jupes de raccordement 38a, 38b ont une zone tronconique 40TR. La zone tronconique 40TR assure un maintien optimal des jupes de raccordement 38a, 38b sur les première et seconde collerettes annulaires 14, 24.

Plusieurs angles différents pourraient d'ailleurs être utilisés.

D'autre part, la première ouverture de communication 34a et la seconde ouverture de communication 34b peuvent avoir des diamètres identiques ou différents. Par exemple, la pièce de raccordement 30 peut faire fonction de réducteur ou d'élargisseur de diamètre, pour le raccordement avec communication de deux contenants 10, 20 et/ou conduits dont le premier orifice 16 et le second orifice 26 ont des diamètres différents.

Selon la réalisation de la figure 2, le premier orifice 16 du premier contenant 10 présente un diamètre inférieur à celui du second orifice 26 du second contenant 20. De la même façon, la première ouverture de communication 34a de la première jupe 38a présente un diamètre inférieur à celui de la seconde ouverture de communication 34b de la seconde jupe 38b. Ainsi, le transfert du produit amont 10a depuis le premier contenant 10 et vers le second contenant 20 ne génère pas de goulot d'étranglement. Cette différence de diamètre est, avantageusement, comprise dans un rapport allant de 1 à 10.

Dans le cas ou là différence de diamètre est suffisant, du fait de la flexibilité de la gaine de raccordement 32, la pièce de raccordement 30 est apte à se trouver lorsqu'elle n'èst pas raccordée aux contenants 10, 20 :
▪ dans une configuration aplatie où les zones en vis-à-vis de la face intérieure de la gaine sont appliquées l'une contre l'autre, et/ou
▪ dans une configuration pliée en accordéon sur elle-même, la première partie extrême libre 32a et la seconde partie extrême libre 32b de la gaine 32 étant rapprochées l'une de l'autre, voir adjacentes l'une à l'autre.

Cela permet notamment de faciliter le conditionnement de ces pièces de raccordement 30 en diminuant considérablement l'espace nécessaire à leur stockage.

Il est toutefois également possible de réaliser une première ouverture de communication 34a et une seconde ouverture de communication 34b présentant un diamètre identique ou sensiblement identique, pour le raccordement avec communication de deux contenants et/ou conduits dont le premier orifice 16 et le second orifice 26 ont un diamètre identique ou sensiblement identique.

D'une façon générale, la première jupe de raccordement 38a èt la seconde jupe de raccordement 38b peuvent d'ailleurs avoir en section axiale une forme identique ou sensiblement identique et une longueur axiale identique ou sensiblement identique, dans un rapport au moins du même ordre que le rapport entre les diamètres de la première ouverture de communication et de la seconde ouverture de communication.

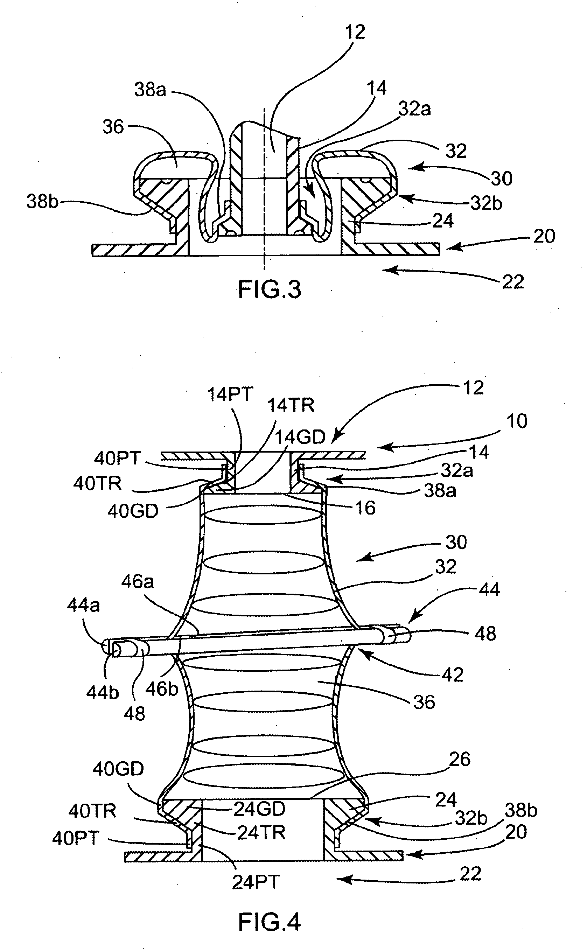

Est maintenant décrite en détail, la réalisation de la figure 3.

Celle-là est identique à la réalisation de la figure 1 à l'exception du fait que la première collerette annulaire 14 du premier contenant 10 est partiellement introduite dans le second espace intérieur 12 défini par la seconde collerette 24 du second contenant 20.

Selon cette réalisation, la gaine de raccordement 32 présente une élasticité telle que les première et seconde jupes 38a, 38b sont maintenus en position contre la première collerette annulaire 14 et la seconde collerette annulaire 24 et assure une étanchéité acceptable dans le domaine biopharmaceutique même lorsque la gaine de raccordement 32 n'est pas tendue.

Selon cette réalisation, la gaine de raccordement 30, du fait de sa souplesse, peut alors se courber pour permettre l'introduction de la première collerette annulaire 14 de plus petit diamètre dans la seconde collerette annulaire 24 de plus grand diamètre.

Est maintenant décrite en détail, la réalisation de la figure 4.

Cette réalisation est également semblable à la réalisation de la figure 1. Toutefois, en l'espèce, la pièce de raccordement 30 ne se trouve plus dans une position ouverte inactive mais dans une configuration fermée d'obturation en une zone transversale d'obturation 42. Dans cette configuration fermée, les zones en vis-à-vis de la face intérieure de la gaine de raccordement 30 sont appliquées l'une contre l'autre et empêchent une communication de part et d'autre de la zone transversale d'obturation 42. Cette réalisation peut être utilisée pour prévenir temporairement le produit amont 10a de⁷ s'écouler au travers de la zone d'obturation 42 jusque dans le second contenant 20. C'est notamment utile lors de la préparation de produits biopharmaceutiques pour gérer les quantités de produits amont 10a à utiliser, la durée de remplissage du second contenant 20, et, par exemple, stopper le transfert du produit amont 10a vers le second contenant 20 lorsque celui-ci est rempli.

Plus particulièrement, dans ce cas de figure l'on utilise un outil d'obturation 44 servant à maintenir les unes contre les autres, les zones de la face intérieure de la gaine de raccordement 30 qui sont en vis-à-vis. L'outil d'obturation 44 permet ainsi, depuis l'extérieur, d'exercer des contraintes sur la face extérieure de la gaine de raccordement 32 de manière à rapprocher, positionner en regard et maintenir les unes contre les autres, certaines zones en vis-à-vis de la face intérieure de cette gaine de raccordement 30.

Selon cette réalisation, cet outil d'obturation 44 comporte deux bras 44a, 44b aptes à se déplacer l'un par rapport à l'autre, chacun d'eux étant doté d'une surface plate d'association 46a, 46b. Ainsi, la gaine de raccordement 32 peut être obstruée par l'outil d'obturation 44 lorsqu'elle est positionnée entre ces deux bras 44a, 44b, que ces deux bras 44a, 44b sont rapprochés l'un de l'autre et que les surfaces plates d'association 46a, 46b sont maintenues l'une contre l'autre.

L'outil d'obturation 44 comporte en outre des moyens de maintien 48 permettant de maintenir de façon structurelle et fonctionnelle ces surfaces plates d'association 46a, 46b en regard l'une de l'autre de manière à empêcher une communication au travers de la zone d'obturation 42.

Selon les proportions de la pièce de raccordement 30, les moyens de maintien 48 assurent une étanchéité - acceptable dans le domaine biopharmaceutique - au niveau des zones de la face intérieure de la gaine de raccordement 32 qui sont jointes.

L'outil d'obturation 44 n'est pas limité à l'exemple décrit et illustré sur la figure 4 mais peut, au contraire, présenter une géométrie différente tout en assurant l'étanchéité de la gaine de raccordement 32 au niveau de la zone d'obturation 42. D'autre part, cette zone d'obturation 42 n'est pas nécessairement transversale mais peut au contraire s'étendre dans un plan oblique par rapport à la section transversale de la gaine de raccordement 32.

L'outil d'obturation 44 pouvant être en outre une simple barrette.

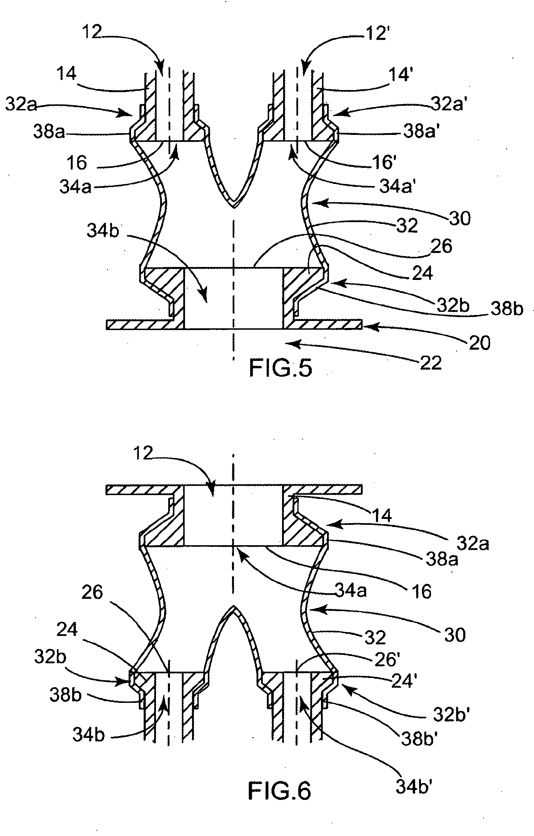

Les figures 5 et 6 sont maintenant décrites de façon détaillée.

La figure 5 illustre un exemple qui diffère de la réalisation de la figure 1 en ce que la pièce de raccordement 30 comporte non pas une première partie extrême libre 32a mais deux premières parties extrêmes libres 32a, 32a'.

Comme précédemment, chacune de ces deux premières parties extrêmes libres 32a, 32a' comporte une première jupe 38a, 38a' et définie une première ouverture de communication 34a, 34a'. Cette réalisation permet d'intégrer dans le second contenant 20 des produits amonts 10a, 10a' - identiques ou différents - provenant respectivement de deux premiers contenants distincts 10, 10'.

La figure 6 illustre, quant à elle, un exemple qui diffère de la réalisation de la figure 1 en ce que la pièce de raccordement 30 comporte non pas une seconde partie extrême libre 32b mais deux secondes parties extrêmes libres 32b, 32b'.

De même que précédemment, chacune de ces deux secondes parties extrêmes libres 32b, 32b' comporte une seconde jupe 38b, 38b' et définie une seconde ouverture de communication 34b, 34b'. Cette réalisation permet d'intégrer - en une étape - dans deux second contenant 20 distincts l'un de l'autre un produit amont 10a provenant du premier contenant 10.

Bien entendu, selon d'autres exemples, la pièce de raccordement 30 peut contenir non plus deux premières parties extrêmes libres 32a, 32a' ou deux secondes parties extrêmes libres 32b, 32b' mais x premières parties extrêmes libres 32a, 32a', 32a", etc. ou x secondes parties extrêmes libres 32b, 32b', 32b" avec x supérieur à deux.

Cet exemple est particulièrement avantageuse pour augmenter la rapidité et l'efficacité de opération de production consistant à réaliser le transfert du (ou des) produit(s) amont(s) 10a provenant du (ou des) premier(s) contenant(s) vers le (ou les) second(s) contenant(s) 20 en vue de former le (ou les) produit(s) aval(s) 20a.

Les premières jupes de raccordement de la pluralité de premières jupes de raccordement peuvent être de même taille ou sensiblement de même taille ou bien de tailles différentes les unes par rapport aux autres.

De façon similaire, les premières sous-gaines de la pluralité de premières sous-gaines sont :
▪ soit de même diamètre et/ou de longueur axiale ou sensiblement de même diamètre et/ou longueur axiale ;
▪ soit de diamètre et/ou de longueur axiale différente les unes des autres.

Comme l'illustrent les figures 1 à 6, la pièce de raccordement 30 selon l'invention peut être raccordée à un premier contenant 10 et à un second contenant 20 en vue de former un ensemble structurel et fonctionnel permettant le transfert du produit amont 10a au travers de la pièce de raccordement 30 et jusqu'au second contenant 20. Le produit amont 10a combiné à d'éventuels autre produit présent dans le second contenant 20 forme ainsi le produit aval 20a.

Mais la pièce de raccordement 30 peut également contenir un moyen fonctionnel tel qu'un organe de disposé au moins partiellement dans l'espace intérieur de raccordement 36.

Ce moyen fonctionnel peut être formé par tout moyen de filtration, moyen de stockage tampon, moyen de connexion, qui, associé de façon structurelle et fonctionnelle à la pièce de raccordement 30 permet le transfert selon un mode opératoire adéquate du produit amont 10a vers le second contenant 20 Selon un mode de réalisation, le moyen fonctionnel peut par exemple former un entonnoir de façon à réaliser un transfert au compte-goutte du produit amont 10a au travers de la pièce de raccordement 30 De façon alternative ou simultanée, le moyen fonctionnel peut également former un filtre permettant de n'autoriser le transfert que d'une partie du produit amont 10a

L'invention n'est pas limitée aux exemples de réalisation précédemment décrits mais s'étend au contraire à tout autre réalisation susceptible d'être mise en oeuvre à partir des connaissances générales de l'homme du métier.

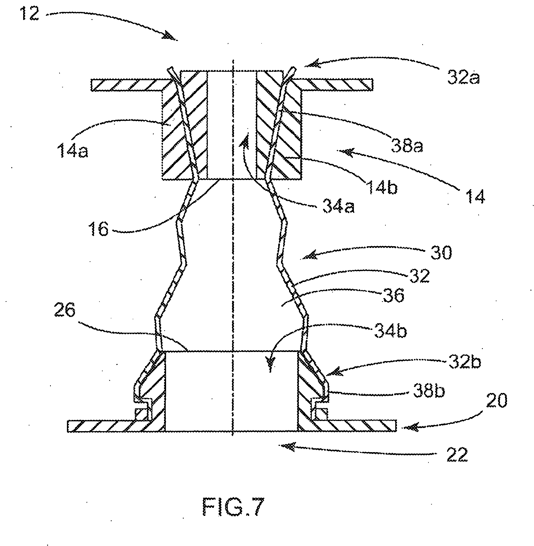

Selon l'exemple représenté sur la figure 7, le premier contenant 10 peut présenter une première collerette annulaire 14 formée par une première partie annulaire 14a de diamètre extérieur tronconique et une seconde partie annulaire 14b également de diamètre extérieur tronconique. La première partie annulaire 14a présente un plus grand diamètre extérieur sur la portion orientée vers le premier espace intérieur 12 que sur la portion orientée vers l'extérieur tandis que la seconde partie annulaire 14b présente un plus grand diamètre extérieur sur la portion orientée vers l'extérieur que sur la portion orientée vers le premier espace intérieur 12.

La première partie annulaire 14a présente en outre un diamètre moyen très légèrement inférieur au diamètre moyen de la seconde partie annulaire 14b. De cette façon, cette première partie annulaire 14a peut s'emboîter dans la seconde partie annulaire 14b de sorte à maintenir la première jupe de raccordement 38a, préalablement enfilée sur la première partie annulaire 14a par élargissement transversal sans perte d'intégrité vis-à-vis du premier orifice 16 du premier contenant 10.

Cette configuration peut d'ailleurs être adaptée soit sur la première collerette annulaire 14 du premier contenant 10, soit sur la seconde collerette annulaire 24 du second contenant 20, ou bien sur les deux collerettes annulaires 14, 24.

## Revendications

1. Pièce de raccordement (30) pour la biopharmacie, apte à raccorder un premier contenant ou conduit et un second contenant ou conduit pour le transfert d'un certain produit biopharmaceutique (10a) du premier contenant ou conduit au second contenant ou conduit, formant un tout :
▪ une gaine de raccordement (32) fermée latéralement sur elle-même, pourvue à sa première partie extrême libre (32a) et à sa seconde partie extrême libre (32b) d'une première ouverture de communication (34a) et d'une seconde ouverture de communication (34b), apte à former et limiter un espace intérieur de raccordement (36), un bord libre d'extrémité délimitant la première ouverture de communication (34a) et un autre bord libre d'extrémité délimitant la seconde ouverture de communication (34b),
▪ à la première partie extrême libre (32a), une première jupe extérieure de raccordement (38a), annulaire et périphérique, dont la face latérale intérieure est apte à venir au contact avec serrage élastique de maintien et étanchéité sur une partie conjuguée de la face extérieure d'une première collerette (14),
▪ à la seconde partie extrême libre (32b), une seconde jupe extérieure de raccordement (38b), annulaire et périphérique, dont la face latérale intérieure est apte à venir au contact avec serrage élastique de maintien et étanchéité sur une partie conjuguée de la face extérieure d'une seconde collerette (24),
- la gaine (32) et les jupes (38a, 38b) étant d'un seul tenant, en un matériau intrinsèquement étanche pour la biopharmacie et ayant une élasticité telle que la pièce de raccordement (30) soit apte à être étirée transversalement à l'endroit de ses deux jupes de raccordement (38a, 38b) et, par son élasticité, à venir au contact avec serrage élastique de maintien et étanchéité sur la partie conjuguée de la face extérieure des deux collerettes (14, 24) sans la nécessité d'une pièce extrinsèque de fixation,
- la pièce de raccordement (30) étant apte, à l'état assemblé avec le premier contenant ou conduit et le second contenant ou conduit et dans une configuration de communication, à permettre le transfert du produit biopharmaceutique (10a) du premier contenant ou conduit au second contenant ou conduit,
**caractérisée par le fait que** :
- la face latérale intérieure de la pièce de raccordement (30) est substantiellement sans aspérité, relief ou rugosité, afin de ne pas freiner le transfert du produit biopharmaceutique (10a) et de ne pas conserver le produit à l'intérieur de la pièce de raccordement (30),
- la gaine de raccordement (32) présente à l'état assemblé et en configuration de communication, une forme générale cylindrique ou tronconique de section transversale au moins sensiblement circulaire ou pseudo-circulaire,
- la face latérale intérieure de l'une et l'autre des jupes (38a, 38b) comporte, successivement au fur et à mesure qu'on se rapproche d'un bord libre d'extrémité correspondant, une zone cylindrique de plus grand diamètre intérieur (40GD) attenante à la gaine (32), une zone tronconique (40TR) et une zone cylindrique de plus petit diamètre intérieur (40PT) attenante au bord libre d'extrémité de la pièce de raccordement (30), la zone tronconique (40TR) étant placée entre la zone cylindrique de plus grand diamètre intérieur (40GD) et la zone cylindrique de plus petit diamètre intérieur (40PT) en s'étendant de l'une à l'autre de ces deux zones cylindriques de la jupe (38a, 38b).

2. Pièce de raccordement (30) selon la revendication 1, **caractérisée par le fait que** la première ouverture (34a) et la seconde ouverture (34b) ont des diamètres différents.

3. Pièce de raccordement (30) selon la revendication 2, **caractérisée par le fait que** la première ouverture (34a) et la seconde (34b) ont des diamètres différents dans un rapport allant de 1 à 10.

4. Pièce de raccordement (30) selon l'une quelconque des revendications 1 à 3, **caractérisée par le fait que**, dans la face latérale extérieure de chaque jupe (38a, 38b) :
- ladite zone cylindrique attenante à la gaine (32) est une zone cylindrique (40GR) de plus grand diamètre, ayant à la fois le plus grand diamètre intérieur et le plus grand diamètre extérieur dans la jupe (38a, 38b) de sorte à présenter une épaisseur radiale constante ;
- ladite zone cylindrique attenante au bord libre d'extrémité est une zone cylindrique (40PT) de plus petit diamètre en ayant à la fois le plus petit diamètre intérieur et le plus petit diamètre extérieur dans la jupe (38a, 38b) de sorte à présenter une épaisseur radiale constante ;
sachant que les jupes (38a, 38b) ont chacune une épaisseur telle que :
- soit la zone tronconique (40TR) est d'épaisseur radiale constante identique à l'épaisseur radiale dans les deux zones cylindriques (40GR, 40PT) entre lesquelles elle s'étend,
- soit la zone cylindrique (40GR) de plus grand diamètre présente une épaisseur radiale plus grande que l'épaisseur radiale dans la zone cylindrique (40PT) de plus petit diamètre.

5. Pièce de raccordement (30) selon l'une quelconque des revendications 1 à 4, **caractérisée par le fait que** la pièce de raccordement (30) est apte à se trouver dans une configuration aplatie d'obturation transversale en au moins une zone transversale d'obturation (42) où les zones en vis-à-vis de la face intérieure de la gaine (30) sont appliquées l'une contre l'autre et empêchent une communication de part et d'autre d'une telle zone transversale d'obturation (42).

6. Pièce de raccordement (30) selon l'une quelconque des revendication 1 à 5, **caractérisée par le fait qu'**elle comporte une unique première et/ou seconde partie extrême libre (32a, 32b) et une unique première et/ou seconde jupe (38a, 38b), la pièce de raccordement (30) étant destinée à être raccordée avec communication à une unique première et/ou seconde collerette (14, 24) d'un unique premier et/ou second contenant (10, 20) ou conduit.

7. Pièce de raccordement (30) selon l'une quelconque des revendications 1 à 6, **caractérisée par le fait que** la gaine (32) et les deux jupes (38a, 38b) sont en un matériau intrinsèquement antistatique ou en un matériau rendu antistatique extrinsèquement.

8. Pièce de raccordement (30) selon l'une quelconque des revendications 1 à 7, **caractérisée par le fait que** la gaine (32) et les deux jupes de raccordement (38a, 38b) sont en silicone ou comprennent du silicone.

9. Pièce de raccordement (30) selon l'une quelconque des revendications 1 à 8, **caractérisée par le fait qu'**elle est à usage unique.

10. Ensemble comprenant une pièce de raccordement (30) selon l'une quelconque des revendications 5 à 9 en ce qu'elles dépendent de la revendication 5, et au moins une pince d'obturation (44) apte à se trouver soit dans une configuration ouverte inactive soit dans une configuration fermée dans laquelle elle est apte à venir se placer autour de la gaine (32) et s'appliquer sur les zones en vis-à-vis de sa face extérieure dans au moins une zone transversale d'obturation (42), de sorte que les zones en vis-à-vis de la face intérieure de la gaine (32) sont appliquées l'une contre l'autre et empêchent une communication de part et d'autre d'une telle zone transversale d'obturation (42).

11. Ensemble comprenant une pièce de raccordement (30) selon l'une quelconque des revendications 1 à 9, et au moins un moyen fonctionnel disposé au moins partiellement dans l'espace intérieur de raccordement (36).

12. Assemblage constitué d'une pièce de raccordement (30) selon l'une quelconque des revendications 1 à 9, d'un premier contenant (10) ou conduit et d'un second contenant (20) ou conduit, tel que :
▪ la face latérale intérieure de la première jupe de raccordement (38a) est au contact avec serrage élastique de maintien et étanchéité sur une partie conjuguée de la face extérieure de la première collerette (14), sans la nécessité d'une pièce extrinsèque de fixation,
▪ et la face latérale intérieure de la seconde jupe de raccordement (38b) est au contact avec serrage élastique de maintien et étanchéité sur une partie conjuguée de la face extérieure de la seconde collerette (24), sans la nécessité d'une pièce extrinsèque de fixation.

13. Assemblage selon la revendication 12, **caractérisé par le fait qu'**il comporte un unique premier et/ou second contenant (10, 20) ou conduit et une pièce de raccordement (30) selon l'une quelconque des revendications 7 à 9 en ce qu'elles dépendent de la revendication 7.

14. Assemblage selon l'une quelconque des revendications 12 et 13, **caractérisé par le fait qu'**il comporte en outre au moins un moyen fonctionnel disposé au moins partiellement dans l'espace intérieur de raccordement (36).

15. Dispositif fonctionnel pour la mise en oeuvre d'un processus biopharmaceutique comprenant au moins un premier contenant (10) ou conduit et un second contenant (20) ou conduit devant être raccordés avec communication, dans lequel le premier contenant (10) ou conduit, le second contenant (20) ou conduit et une pièce de raccordement (30) selon l'une quelconque des revendications 1 à 9 forment un assemblage (2) selon l'une quelconque des revendications 12 et 13.

16. Procédé pour raccorder avec communication en biopharmacie, deux contenants (10, 20) et/ou conduits comportant, respectivement, une première collerette annulaire (14) résistante et une seconde collerette annulaire (24) résistante limitant un premier orifice (16) et un second orifice (26), **caractérisé par le fait que** :
▪ on dispose du premier contenant (10) ou conduit et du second contenant (20) ou conduit,
▪ on dispose d'une pièce de raccordement (30) selon l'une quelconque des revendications 1 à 9 se trouvant à l'état désassemblé,
▪ on étire transversalement la pièce de raccordement (30) à l'endroit de ses deux jupes de raccordement (38a, 38b) jusqu'à être élargie transversalement pour pouvoir être enfilée sur les deux collerettes (14, 24),
▪ puis, on laisse la pièce de raccordement (30) venir, par son élasticité, au contact avec serrage élastique de maintien et étanchéité sur la partie conjuguée de la face extérieure des deux collerettes (14, 24) et être ainsi amenée à l'état assemblé, sans la nécessité d'une pièce extrinsèque de fixation.

## Patentansprüche

1. Verbindungsstück (30) für die Biopharmazie, welches in der Lage ist, einen ersten Behälter oder eine erste Leitung und einen zweiten Behälter oder eine zweite Leitung für den Transfer eines bestimmten biopharmazeutischen Produkts (10a) von dem ersten Behälter oder der ersten Leitung zu dem zweiten Behälter oder der zweiten Leitung zu verbinden, wobei eine Gesamtheit bilden:
- eine Verbindungshülse (32), welche lateral an sich selbst geschlossen ist, welche an ihrem ersten freien Endteil (32a) und an ihrem zweiten freien Endteil (32b) mit einer ersten Kommunikationsöffnung (34a) und einer zweiten Kommunikationsöffnung (34b) versehen ist, welche in der Lage ist, einen Verbindungs-Innenraum (36) zu bilden und zu begrenzen, wobei ein freier Endrand die erste Kommunikationsöffnung (34a) begrenzt und ein anderer freier Endrand die zweite Kommunikationsöffnung (34b) begrenzt,
- an dem ersten freien Endteil (32a) eine erste äußere ringförmige und umfängliche Verbindungsschulter (38a), deren innere laterale Fläche dazu in der Lage ist, in Kontakt mit elastischem Klemmen für ein Halten und Abdichten an einem Teil zu kommen, welcher mit der äußeren Fläche eines ersten Kragens (14) gekoppelt ist,
- an dem zweiten freien Endteil (32b) eine zweite äußere ringförmige und umfängliche Verbindungsschulter (38b), deren innere laterale Fläche dazu in der Lage ist, in Kontakt mit elastischem Klemmen für ein Halten und Abdichten an einem Teil zu kommen, welcher mit der äußeren Fläche eines zweiten Kragens (24) gekoppelt ist,
- wobei die Hülse (32) und die Schultern (38a, 38b) einstückig und aus einem für die Biopharmazie intrinsisch dichten Material sind und eine derartige Elastizität aufweisen, dass das Verbindungsstück (30) in der Lage ist, transversal an der Position seiner beiden Verbindungsschultern (38a, 38b) gestreckt zu werden und durch seine Elastizität in Kontakt mit elastischem Klemmen für ein Halten und Abdichten an dem Teil zu kommen, welcher mit der äußeren Fläche der beiden Krägen (14, 24) gekoppelt ist, ohne die Notwendigkeit eines extrinsischen Befestigungsstücks,
- wobei das Verbindungsstück (30) in der Lage ist, in mit dem ersten Behälter oder der ersten Leitung und dem zweiten Behälter oder der zweiten Leitung montierten Zustand und in einer Kommunikationskonfiguration den Transfer des biopharmazeutischen Produkts (10a) von dem ersten Behälter oder der ersten Leitung zu dem zweiten Behälter oder der zweiten Leitung zu erlauben,
**dadurch gekennzeichnet, dass**:
- die innere laterale Fläche des Verbindungsstücks (30) im Wesentlichen ohne Unebenheit, Relief oder Rauheit ist, um so den Transfer des biopharmazeutischen Produkts (10a) nicht zu bremsen und das Produkt nicht im Innenraum des Verbindungsstücks (30) zu halten,
- die Verbindungshülse (32) im montierten Zustand und in Kommunikationskonfiguration eine im Wesentlichen zylindrische oder kegelförmige Form mit einem transversalen Querschnitt aufweist, welcher wenigstens näherungsweise kreisförmig oder pseudo-kreisförmig ist,
- die innere laterale Fläche von der einen und der anderen der Schultern (38a, 38b) sukzessive nach und nach bei einer Annäherung an einen entsprechenden freien Endrand eine zylindrische Zone mit größtem Innendurchmesser (40GD) angrenzend an die Hülse (32), eine konische Zone (40TR) und eine zylindrische Zone mit kleinstem Innendurchmesser (40PT) angrenzend an den freien Endrand des Verbindungsstücks (30) umfasst, wobei die konische Zone (40TR) zwischen der zylindrischen Zone mit größtem Innendurchmesser (40GD) und der zylindrischen Zone mit kleinstem Innendurchmesser (40PT) platziert ist, indem sie sich von der einen zu der anderen der beiden zylindrischen Zonen der Schulter (38a, 38b) ausbreitet.

2. Verbindungsstück (30) nach Anspruch 1, **dadurch gekennzeichnet, dass** die erste Öffnung (34a) und die zweite Öffnung (34b) unterschiedliche Durchmesser aufweisen.

3. Verbindungsstück (30) nach Anspruch 2, **dadurch gekennzeichnet, dass** die erste Öffnung (34a) und die zweite (34b) unterschiedliche Durchmesser in einem Verhältnis aufweisen, welches von 1 bis 10 beträgt.

4. Verbindungsstück (30) nach einem der Ansprüche 1 bis 3, **dadurch gekennzeichnet, dass** in der äußeren lateralen Fläche von jeder Schulter (38a, 38b):
- die an die Hülse (32) angrenzende zylindrische Zone eine zylindrische Zone (40GR) mit größtem Durchmesser ist, welche gleichzeitig den größten Innendurchmesser und den größten Außendurchmesser in der Schulter (38a, 38b) derart aufweist, dass sie eine konstante radiale Dicke aufweist;
- die an den freien Endrand angrenzende zylindrische Zone eine zylindrische Zone (40PT) mit kleinstem Durchmesser ist, indem sie gleichzeitig den kleinsten Innendurchmesser und den kleinsten Außendurchmesser in der Schulter (38a, 38b) derart aufweist, dass sie eine konstante radiale Dicke aufweist;
wobei die Schultern (38a, 38b) jeweils eine derartige Dicke aufweisen, dass:
- entweder die konische Zone (40TR) von einer identischen konstanten radialen Dicke wie die radiale Dicke in den beiden zylindrischen Zonen (40GR, 40PT) ist, zwischen welchen sie sich erstreckt,
- oder die zylindrische Zone (40GR) mit größtem Durchmesser eine größere radiale Dicke als die radiale Dicke in der zylindrischen Zone (40PT) mit kleinstem Durchmesser aufweist.

5. Verbindungsstück (30) nach einem der Ansprüche 1 bis 4, **dadurch gekennzeichnet, dass** das Verbindungsstück (30) dazu in der Lage ist, sich in einer abgeflachten Konfiguration für ein transversales Verschließen in wenigstens einer transversalen Verschlusszone (42) zu befinden, in welcher die Zonen gegenüber der inneren Fläche der Hülse (30) die eine gegen die andere anliegen und eine Kommunikation beiderseits einer derartigen transversalen Verschlusszone (42) verhindern.

6. Verbindungsstück (30) nach einem der Ansprüche 1 bis 5, **dadurch gekennzeichnet, dass** es einen einzelnen ersten und/oder zweiten freien Endteil (32a, 32b) und eine einzelne erste und/oder zweite Schulter (38a, 38b) umfasst, wobei das Verbindungsstück (30) dazu vorgesehen ist, mit Kommunikation an einem einzelnen ersten und/oder zweiten Kragen (14, 24) eines einzelnen ersten und/oder zweiten Behälters (10, 20) oder einer einzelnen ersten und/oder zweiten Leitung angeschlossen zu sein.

7. Verbindungsstück (30) nach einem der Ansprüche 1 bis 6, **dadurch gekennzeichnet, dass** die Hülse (32) und die beiden Schultern (38a, 38b) aus einem intrinsisch antistatischen Material oder aus einem Material bestehen, welches extrinsisch antistatisch gemacht worden ist.

8. Verbindungsstück (30) nach einem der Ansprüche 1 bis 7, **dadurch gekennzeichnet, dass** die Hülse (32) und die beiden Verbindungsschultern (38a, 38b) aus Silikon bestehen oder Silikon umfassen.

9. Verbindungsstück (30) nach einem der Ansprüche 1 bis 7, **dadurch gekennzeichnet, dass** es für einen einmaligen Gebrauch ist.

10. Anordnung, umfassend ein Verbindungsstück (30) nach einem der Ansprüche 5 bis 9, wenn sie von Anspruch 5 abhängen, und wenigstens ein Verschlussstück (44), welches in der Lage ist, sich entweder in einer inaktiven offenen Konfiguration oder in einer geschlossenen Konfiguration zu befinden, in welcher es in der Lage ist, sich um die Hülse (32) herum platzieren und sich auf die Zonen gegenüber seiner äußeren Fläche in wenigstens einer transversalen Verschlusszone (42) anliegen zu gehen, so dass die Zonen gegenüber der inneren Fläche der Hülse (32) die eine gegen die andere anliegen und eine Kommunikation beiderseits einer derartigen transversalen Verschlusszone (42) verhindern.

11. Anordnung, umfassend ein Verbindungsstück (30) nach einem der Ansprüche 1 bis 9 und wenigstens ein funktionelles Mittel, welches wenigstens teilweise in dem Verbindungs-Innenraum (36) angeordnet ist.

12. Baugruppe, gebildet aus einem Verbindungsstück (30) nach einem der Ansprüche 1 bis 9, einem ersten Behälter (10) oder einer ersten Leitung und einem zweiten Behälter (20) oder einer zweiten Leitung, so dass:
- die innere laterale Fläche der ersten Verbindungsschulter (38a) in Kontakt mit elastischem Klemmen für ein Halten und Abdichten an einem Teil ist, welcher mit der äußeren Fläche eines ersten Kragens (14) gekoppelt ist, ohne die Notwendigkeit eines extrinsischen Befestigungsteils,
- und die innere laterale Fläche der zweiten Verbindungsschulter (38b) in Kontakt mit elastischem Klemmen für ein Halten und Abdichten an einem Teil ist, welcher mit der äußeren Fläche eines zweiten Kragens (24) gekoppelt ist, ohne die Notwendigkeit eines extrinsischen Befestigungsteils.

13. Baugruppe nach Anspruch 12, **dadurch gekennzeichnet, dass** sie einen ersten und/oder zweiten Behälter (10, 20) oder eine erste und/oder zweite Leitung und ein Verbindungsstück (30) nach einem der Ansprüche 7 bis 9, wenn sie von Anspruch 7 abhängen, umfasst.

14. Baugruppe nach einem der Ansprüche 12 und 13, **dadurch gekennzeichnet, dass** sie ferner wenigstens ein funktionelles Mittel umfasst, welches wenigstens teilweise in dem Verbindungs-Innenraum (36) angeordnet ist.

15. Funktionelle Vorrichtung zum Durchführen eines biopharmazeutischen Prozesses, umfassend wenigstens einen ersten Behälter (10) oder eine erste Leitung und einen zweiten Behälter (20) oder eine zweite Leitung bevor sie mit Kommunikation verbunden werden, wobei der erste Behälter (10) oder die erste Leitung, der zweite Behälter (20) oder die zweite Leitung und ein Verbindungsstück (30) nach einem der Ansprüche 1 bis 9 eine Baugruppe (2) nach einem der Ansprüche 12 und 13 bilden.

16. Verfahren zum Verbinden mit Kommunikation in der Biopharmazie von zwei Behältern (10, 20) und/oder Leitungen, umfassend jeweils einen ersten widerstandsfähigen ringförmigen Kragen (14) und einen zweiten widerstandsfähigen ringförmigen Kragen (24), welche eine erste Öffnung (16) und eine zweite Öffnung (26) begrenzen, **gekennzeichnet durch**:
- Anordnen des ersten Behälters (10) oder der ersten Leitung und des zweiten Behälters (20) oder der zweiten Leitung,
- Anordnen eines Verbindungsstücks (30) nach einem der Ansprüche 1 bis 9 sich in dem demontierten Zustand befindend,
- transversales Ziehen des Verbindungsstücks (30) an der Position seiner beiden Verbindungsschultern (38a, 38b), bis es transversal gestreckt ist, um auf die beiden Krägen (14, 24) gesteckt werden zu können,
- danach in Kontakt mit elastischem Klemmen für ein Halten und Abdichten an einem Teil, welches mit der äußeren Fläche der beiden Krägen (14, 24) gekoppelt ist, kommen lassen des Verbindungsstücks (30) durch seine Elastizität, und somit Überführen in den montierten Zustand ohne die Notwendigkeit eines extrinsischen Befestigungsstücks.

## Claims

1. Connecting part (30) for biopharmacy, capable of connecting a first container or conduit and a second container or conduit for the transfer of a certain biopharmaceutical product (10a) from the first container or conduit to the second container or conduit, forming a whole:
▪ A connecting sheath (32) closed laterally on itself, provided at its first free end part (32a) and at its second free end part (32b) with a first communication opening (34a) and a second communication opening (34b), capable of forming and limiting an inner connecting space (36), a free end edge delimiting the first communication opening (34a) and another free end edge delimiting the second communication opening (34b),
▪ At the first free end part (32a), a first annular and peripheral outer connecting skirt (38a), whose inner lateral face is capable of coming into contact with elastic tightening for holding and sealing on a part mated to the outer face of a first collar (14),
▪ At the second free end part (32b), a second annular and peripheral outer connecting skirt (38b), whose inner lateral face is capable of coming into contact with elastic tightening for holding and sealing on a part mated to the outer face of a second collar (24),
▪ The sheath (32) and the skirts (38a, 38b) being integral, made of an inherently airtight material for biopharmacy and having an elasticity such that the connecting part (30) is capable of being stretched transversely at the site of its two skirts (38a, 38b) and, by its elasticity, coming into contact with elastic tightening for holding and sealing on the part mated to the outer face of the collars (14, 24), without the necessity for an extrinsic attachment part,
▪ The connecting part (30) being capable of, in the assembled state with the first container or conduit and the second container or conduit and in a communication configuration, allowing transfer of the biopharmaceutical product (10a) from the first container or conduit to the second container or conduit,
**characterized by the fact that:**
▪ The inner face of the connecting part (30) is essentially without rough spots, reliefs or roughness so as not to slow down the transfer of biopharmaceutical product (10a) and not to keep the product inside the connecting part (30),
▪ The connecting sheath (32) has, in the assembled state and in a communication configuration, a general cylindrical or tapered shape with a transverse cross-section that is at least approximately circular or pseudo-circular,
▪ The inner lateral faces of one and the other of the skirts (38a, 38b) comprises, successively with increasing proximity to a corresponding free end edge, a cylindrical zone of larger inside diameter (40GD) adjacent to the sheath (32), a tapering zone (40TR) and a cylindrical zone of smaller inside diameter (40PT) adjacent to the free end edge (32a, 32b) of the connecting part (30), the tapered zone (40TR) being placed between the cylindrical zone of larger inside diameter (40GD) and the cylindrical zone of smaller inside diameter (40PT), extending between these two cylindrical zones of the skirt (38a, 38b).

2. Connecting part (30) according to Claim 1, wherein the first opening (34a) and the second opening (34b) have different diameters.

3. Connecting part (30) according to Claim 2, wherein the first opening (34a) and the second opening (34b) have diameters in a ratio that ranges from 1 to 10.

4. Connecting part (30) according to any of Claims 1 to 3, wherein in the outer lateral wall of each skirt (38a, 38b):
- said cylindrical zone adjacent to the sheath (32) is a cylindrical zone (40GR) of greater diameter, having both the largest inner diameter and the largest outer diameter in the skirt (38a, 38b) so as to present a constant radial thickness;
- said cylindrical zone adjacent the free end edge is a cylindrical zone (40PT) of smaller diameter having both the smallest inner diameter and the smallest outer diameter in the skirt (38a, 38b) so as to present a constant radial thickness;
knowing that the skirts (38a, 38b) each have a thickness such that:
- either the tapering zone (40TR) (40TR) is of constant radial thickness identical to the radial thickness in the two cylindrical zones (40GR, 40PT) between which it extends,
- or the cylindrical zone (40GR) of greater diameter has a radial thickness greater than the radial thickness in the cylindrical zone (40PT) of smaller diameter.

5. Connecting part (30) according to any of Claims 1 to 4, wherein the connecting part (30) is capable of being located in a flattened transverse blocking configuration in at least one transverse blocking zone (42) where the zones opposite the inner face of the sheath (30) are applied against one another and prevent communication on both sides of such a transverse blocking zone (42).

6. Connecting part (30) according to any of Claims 1 to 5, wherein it comprises a single first and/or second free end part (32a, 32b) and a single first and/or second skirt (38a, 38b), with the connecting part (30) being designed to be connected with communication to a single first and/or second collar (14, 24) of a single first and/or second container (10, 20) or conduit.

7. Connecting part (30) according to any of Claims 1 to 6, wherein the sheath (32) and the two skirts (38a, 38b) are made of an inherently anti-static material or a material that is made extrinsically anti-static.

8. Connecting part (30) according to any of Claims 1 to 7, wherein the sheath (32) and the two skirts (38a, 38b) are made of silicone or comprise silicone.

9. Connecting part (30) according to any of Claims 1 to 8, wherein it is disposable.

10. Unit comprising a connecting part (30) according to any of Claims 5 to 9, in that they depend on Claim 5, and at least one blocking clamp (44) that can be found either in an inactive open configuration or in a closed configuration in which it is able to come to be placed around the sheath (32) and to be applied to zones opposite its outer face in at least one transverse blocking zone (42), in such a way that the zones opposite the inner face of the sheath (32) are applied against one another and prevent communication on both sides of such a transverse blocking zone (42).

11. Unit comprising a connecting part (30) according to any of Claims 1 to 9, and at least one functional means that is placed at least partially in the inner connecting space (36).

12. Assembly that consists of a connecting part (30) according to any of Claims 1 to 9, a first container (10) or conduit, and a second container (20) or conduit, such that:
▪ The inner lateral face of the first connecting skirt (38a) is in contact with elastic tightening for holding and sealing on a part mated to the outer face of the first collar (14), without the necessity for an extrinsic attachment part,
▪ And the inner lateral face of the second connecting skirt (38b) is in contact with elastic tightening for holding and sealing on a part mated to the outer face of the second collar (24), without the necessity for an extrinsic attachment part.

13. Assembly according to Claim 12, wherein it comprises a single first and/or second container (10, 20) or conduit and a connecting part (30) according to any of Claims 7 to 9, in that they depend on Claim 7.

14. Assembly according to any of Claims 12 and 13, wherein it also comprises at least one functional means placed at least partially in the inner connecting space (36).

15. Functional device for the implementation of a biopharmaceutical process comprising at least a first container (10) or conduit and a second container (20) or conduit having to be connected with communication, in which the first container (10) or conduit, the second container (20) or conduit, and a connecting part (30) according to any of Claims 1 to 10 form an assembly (2) according to any of Claims 12 and 13.

16. Process for connecting with communication in biopharmacy two containers (10, 20) and/or conduits comprising, respectively, a first resistant annular collar (14) and second resistant annular collar (24) limiting a first opening (16) and a second opening (26), wherein:
▪ There are the first container (10) or conduit and the second container (20) or conduit,
▪ There is a connecting part (30) according to any of Claims 1 to 11 that is in the disassembled state,
▪ The connecting part (30) is stretched transversely at the site of its two connecting skirts (38a, 38b) until being broadened transversely to be able to be threaded onto the two collars (14, 24),
▪ Then, the connecting part (30) is allowed to come, by its elasticity, into contact with elastic tightening for holding and sealing on the part mated to the outer face of the two collars (14, 24) and thus to be brought into the assembled state, without the necessity for an extrinsic attachment part.
